# EUROPÄISCHE PATENTSCHRIFT

(11) **EP 3 217 927 B1**
(45) Veröffentlichungstag und Bekanntmachung des Hinweises auf die Patenterteilung: **08.04.2020**
(21) Anmeldenummer: 15797863.6
(22) Anmeldetag: 10.11.2015
(51) Int. Cl.: A61F 2/915

(54) **STENTPROTHESE**
STENT
STENT PROTHÉTIQUE

(30) Priorität: 11.11.2014 DE 102014016588
(43) Veröffentlichungstag der Anmeldung: 20.09.2017
(73) Patentinhaber: medicut Stent Technology GmbH, 75179 Pforzheim (DE)
(72) Erfinder: JUNG, Johannes, 75181 Pforzheim (DE); FALLAHI, Aryan, 75177 Pforzheim (DE)
(74) Vertreter: Winter, Brandl, Fürniss, Hübner, Röss, Kaiser, Polte - Partnerschaft mbB
(86) Internationale Anmeldenummer: PCT/EP2015/025081
(87) Internationale Veröffentlichungsnummer: WO 2016/074799

(56) Entgegenhaltungen:
- EP-A1- 2 438 872
- WO-A1-01/26583
- WO-A1-97/25000
- FR-A1- 2 768 919
- US-A- 5 843 175
- US-A- 5 938 697
- US-A1- 2009 248 132

## Beschreibung

Die Erfindung betrifft einen Stent für den Einsatz in röhrenförmigen Hohlorganen mit einem durch eine Wand begrenzten, durchgängigen, innenliegenden, tubulären bzw. zylinderförmigen Hohlraum, wobei die Wand um eine in longitudinaler Richtung verlaufende Achse herum tubulär bzw. zylinderförmig ausgebildet ist und eine die Wand umlaufenden Struktur aufweist. Die Struktur ist hierbei aus Elementen gebildet und die Elemente sind über Verbindungsstellen derart fest verbunden, dass eine einstückige, tubuläre bzw. zylinderförmige Wandstruktur entsteht.

Stents werden unter anderem als Prothesen zur Erweiterung, Offenhaltung und Stabilisierung in röhrenförmige Hohlorgane wie beispielsweise Blutgefäße eingesetzt. In der Regel weisen derartige Stents hierbei Gitter oder spiralförmige Strukturen aus Materialstegen auf. Hierbei sind meist zwischen den Materialstegen materialfreie Bereiche ausgebildet, die es ermöglichen, dass Gewebe am Implantationsort einwachsen kann. Derartige Stents sind beispielsweise in der Druckschrift DE A 197 46 88 beschrieben.

Üblicherweise werden derartige Prothesen unter anderem zur Therapie von Stenosen eingesetzt, wobei mittels der Prothesen die Wand eines Gefäßes, beispielsweise einer Arterie, derart erweitert und offengehalten wird, dass ein ausreichender Durchfluss durch das Gefäß gewährleistet ist. Die Prothesen weisen dabei im Allgemeinen sehr geringe Wandstärken auf, wobei jedoch eine gewisse Radialfestigkeit gegeben sein muss, damit die Form der Prothese nach Implantation in ein Gefäß in der gewünschten Form erhalten bleibt. Neben einer radialen Festigkeit sollte die Prothese außerdem eine ausreichende Biegefestigkeit aufweisen, die es ermöglicht, dass sich die Prothese der teilweise gekrümmten Form und den Bewegungen des Gefäßabschnittes, in dem die Prothese implantiert ist, hinreichend anpassen kann.

Bei Prothesen nach dem Stand der Technik besteht das Problem, dass in den biegsamen Gefäßbereichen, wie beispielsweise im Bereich der Gelenke, eine implantierte Prothese erheblichen Stauch-, Zug- und Drehbewegungen ausgesetzt ist. Zusätzlich wirken im Bereich der Biegung auch Reibungskräfte auf die Intima der Blutgefäße ein. Dabei werden durch die genannten Stauch-, Zug- und Drehbewegungen die Materialstege der Prothese geschwächt, so dass es zu Brüchen und zu einzelnen Ablösungen von Prothese-Fragmenten kommen kann. Dies kann unter anderem zu Verletzungen der Gefäßwand, zu Restenosen, z. B. durch Narbengewebsbildung an der Gefäßwand, oder sogar zu einer Ausbildung eines Aneurysma führen. Gegebenenfalls können hierbei auch Embolien ausgelöst werden.

Üblicherweise wird die Prothese in einem komprimierten Zustand bei der Implantation in das Gefäß eingeführt und anschließend an dem zu behandelnden Ort expandiert, was in der Regel mittels eines Ballonkatheters erfolgt. Ein Ballonkatheter wird ebenfalls meist auch zur Positionierung der Prothese im Gefäß verwendet. Jedoch ist es auch möglich, eine selbst expandierende Prothese zu verwenden, welche in einem komprimierten Zustand mittels eines Katheters in das Gefäß eingeführt wird und anschließend an der gewünschten Stelle des Gefäßes freigesetzt wird, was bei einer gleichzeitigen Expansion der Prothese realisiert wird.

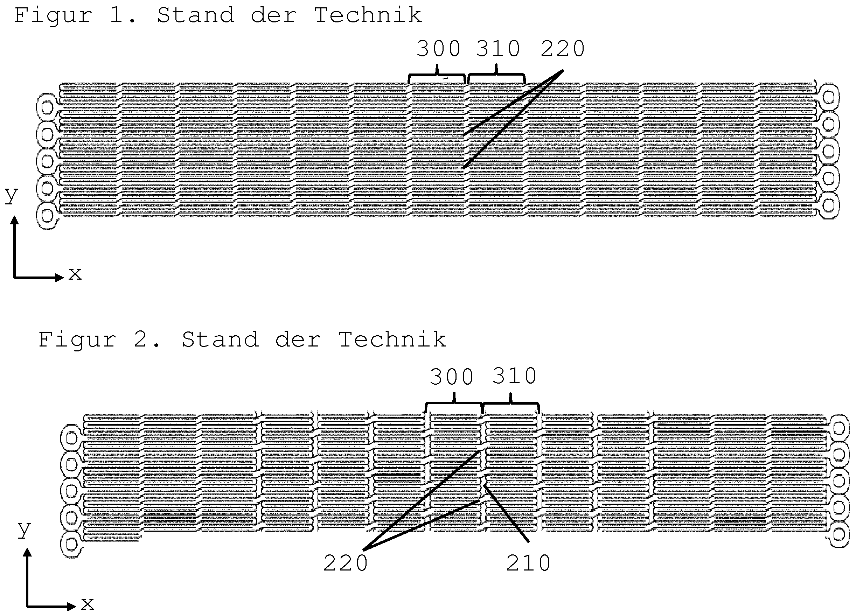

Aus dem Stand der Technik sind zwei grundlegende Stent-Techniken bzw. Strukturen bekannt, nämlich das sogenannte Open Cell Design, wie es in der den Stand der Technik darstellenden Fig. 1 dargestellt ist, und das Closed Cell Design, wie es in der den Stand der Technik beschreibenden Fig. 2 dargestellt ist. Beim sogenannten Open Cell Design handelt es sich um ein flexibles Design, während es sich beim Closed Cell Design um ein relativ starres Design des Stents handelt.

Üblicherweise ist die Struktur dieser Stents im Wesentlichen gitterförmig, wobei dieses Gitter unterschiedlichste Ausgestaltungen haben kann. Beispielsweise ist die Struktur des starren Designs rautenförmig oder rhomboid, wobei hier die Eckpunkte eines Rhomboids mit denen von anderen Rhomboiden verbunden sind. Nachteilig an dieser starren Struktur ist, dass diese beim Biegen einknickt, wodurch der über die Knickstelle strömende Flüssigkeitsstrom (z. B. Blutstrom) zu Verwirbelungen neigt, wobei sich statt einer gewünschten laminaren Strömung eine turbulente Strömung im Knickbereich ausbildet. Diese turbulente Strömung führt gewöhnlich zu unerwünschten Ablagerungen, gegebenenfalls einem Zusammenballen von Erythrozyten, mit möglichen Folgen einer Neointimahyperplasie und Arterosklerose bis hin zu symptomatischen Restenosen. Ein weiterer Nachteil dieses Designs ist, dass bei Aufdehnung des Stents in radialer Richtung die sog. Verbinder an den Rhomboid-(Rauten-)Eckpunkten einreißen können. Ein Vorteil dieses starren Designs ist jedoch, dass ein Stent, wenn er falsch oder ungenau innerhalb eines Gefäßes abgelegt wurde, wieder in den Katheter bzw. die Schleuse eines Katheters einziehbar ist. Dies ist beim sogenannten flexiblen oder Open Cell Design nicht möglich, da hier der Stent beim Wiedereinziehen in den Katheter bzw. die Schleuse bricht. Dies hängt insbesondere auch damit zusammen, dass bei dem flexiblen Design miteinander verbundenen Stent-Elemente mittels sog. Verbinder zusammengehalten werden, wobei die Stent-Elemente dabei jedoch immer durch mindestens eine nicht verbundene Stelle aneinander gekettet vorliegen. Der Vorteil des flexiblen Designs besteht jedoch darin, dass in Biegestellen ein Einknicken des gesamten Stents großteils verhindert wird. Auch ist die Radialfestigkeit des flexiblen Stentdesigns, d.h. die Festigkeit, die der radial nach innen gerichteten Kraft des Gefäßes entgegenwirkt, im Vergleich zu der des starren Stentdesigns geringer.

Aus dem Stand der Technik sind weitere Stents, welche aus ineinander verwobenen Drähten gebildet werden, bekannt. Der Vorteil dieser Stents liegt darin, dass diese flexibel sind, d. h. in Biegestellen nicht einknicken und dabei eine hohe Radialfestigkeit aufweisen. Solche Stents sind beispielsweise in der Druckschrift US 2012/0330398 A1 beschrieben. Nachteilig an derartigen Stents ist jedoch, dass der geflochtene Draht Kreuzungspunkte aufweist, welche die Gefäßinnenwände schädigen können. Da diese Kreuzungspunkte nicht glatt sind, können hierdurch leicht, nicht laminare Turbulenzen auftreten, was zu unerwünschten Ablagerungen z. B. durch Aggregation von Erythrozyten führen kann, wodurch sich gegebenenfalls Restenosen entwickeln können.

Weiter wird in DE 196 53 708 A1 einen Stent beschrieben, welcher eine röhrenförmige/zylindrische Grundform mit zahlreichen Durchbrüchen aufweist, welche von expansiblen Strukturelementen umschlossen sind, die die Form von gestauchten Ringen aufweisen. Diese expansiblen Elemente werden durch umlaufende schmale stegartige Bereiche mit rechteckigem Querschnitt gebildet und zeichnen sich dadurch aus, dass sie eine Ausnehmung ringförmig umschließen. Die ein expansibles Element umschließenden stegartigen Bereiche sind dabei mehrfach s-förmig geschwungen ausgebildet. Sie umschließen jeweils eine Ausnehmung in der Weise, dass die sich in tangentialer Richtung benachbart gegenüberliegenden, stegartigen Bereiche derselben oder einer benachbarten Ausnehmung spiegelsymmetrisch angeordnet sind. Zwischen den in axialer (Läng-) und in tangialer (Quer-)Richtung benachbart auf der Mantelfläche des Stents angeordneten Ausnehmungen sind Verbindungsbereiche/Verbinder vorgesehen, welche die jeweiligen expandierenden Bereiche mechanisch miteinander koppeln. Diese Verbindungsbereiche/Verbinder sind hier kreuzförmig ausgebildet, wobei die einzelnen Arme des Kreuzes die Form von Bogenstücken aufweisen.

Eine ähnliche Struktur wird auch in EP 0 903 123 A1 beschrieben. Hier wird ein Stent offenbart, welcher entlang einer longitudinalen Achse eine Vielzahl von longitudinal angeordneten Bändern aufweist, wobei jedes Band die Form einer kontinuierlichen Welle aufweist, welche entlang einer Segmentlinie parallel zur longitudinalen Achse verläuft. Dabei hält eine Vielzahl von Verbindungselementen/Verbindern die Bänder in einer tubulären Struktur zusammen. Dabei ist ein solches longitudinales Band des Stents an einer Vielzahl von periodisch auftretenden Stellen mit einem benachbarten Band verbunden.

FR 2 768 919 A1 beschreibt einen Stent mit einstückiger tubulärer Struktur. Hierbei ist es so, dass gewählte Linien den Stent umlaufen und diese Linien jeweils mittels Verbindern sog. "tronçons intermediaires" bzw. "barrettes de liaison" zusammengehalten werden und so die einstückige Struktur des Stents bilden (vgl. FIG. 7 und FIG. 8 von D1).

In WO 01/26583 A1 wird ein Stent beschrieben, welcher aus einer Vielzahl von Zellen aufgebaut ist, die wiederum radial um den Stent verlaufen. Hierbei ist es so, dass die Zellen aus einer Vielzahl von Streben, welche miteinander verbunden sind, gebildet werden. In Abbildung 13 von D2 im Bereich 54i liegt auch hier ein Verbinder vor, welcher die einzelnen Zellen miteinander verbindet.

US 5,843,175 A1 beschreibt einen Stent mit einer zylindrischer Konfiguration aus Streben welcher bei Operationen in Arterien eingesetzt werden kann. Hierbei werden einzelne Streben mittels Verbindern (eng. "links", Bezugszeichen 180) zusammen gehalten.

US 5,938,697 A1 beschreibt einen kegelförmigen tubulären Stent zum Einsatz in Gefäßen wie Arterien. Der Stent gemäß dieser Druckschrift besteht hierbei immer aus offenen Bereichen, welche mittels Verbindern zusammengehalten werden als auch aus geschlossenen Bereichen.

In EP 2 438 872 A1 wird einen Stent offenbart mit einem Grundkörper aus mindestens zwei Stegen und mindestens zwei sich in axialer Richtung gegenüberliegenden Umlenkstellen, an welche ein Verstellmittel angesetzt werden kann, um den Grundkörper in Umfangrichtung aufzuweiten.

Aufgrund der strukturellen Ausbildung dieser o.g. Stents im Bereich der Verbinder, welche zum Teil Bogenstücke aufweisen, kann es bei einer Aufdehnung oder Expansion in diesem Bereich zu einem Einreißen der Struktur kommen. Weiter können auch Knicke bei Biegung des Stents im Bereich der Verbinder aufgrund der Länge dieser Verbinder auftreten, welche die Gefäßwände schädigen können, bzw. dies kann, wie bereits oben beschrieben, zu nichtlaminaren Turbulenzen führen.

Eine Aufgabe der Erfindung besteht darin, die Nachteile des Stands der Technik zu überwinden, insbesondere soll der erfindungsgemäße Stent beim Biegen keinen Knick ausbilden. Darüber hinaus soll der Stent wieder ohne zu brechen in die Schleuse eines Katheters eingezogen werden können, und glatte Ober-/Innenflächen besitzen. Des Weiteren soll eine Erhöhung der radial nach außen wirkenden Kräfte des Stents erreicht werden.

Die Aufgabe wird durch einen erfindungsgemäßen Stent gemäß Anspruch 1 gelöst. In den Unteransprüchen sind vorteilhafte Weiterbildungen und bevorzugte Ausführungsformen angegeben.

Erfindungsgemäß wird ein Stent zur Verfügung gestellt, mit einem durch eine Wand begrenzten, durchgängigen, innenliegenden, im Wesentlichen tubulären bzw. zylinderförmigen Hohlraum, wobei die Wand um eine in longitudinaler Richtung verlaufende Achse herum im Wesentlichen tubulär bzw. zylinderförmig ausgebildet ist. Die Wand weist dabei eine umlaufende Struktur auf, wobei die Struktur aus Elementen gebildet ist. Erfindungsgemäß sind die Elemente des Stents aus Schlaufen gebildet, welche im Wesentlichen in radialer Richtung um die longitudinale Achse herum angeordnet sind und alternierend angeordnete, im Wesentlichen zur longitudinalen Richtung parallel verlaufende Schlaufenköpfe und Schlaufentäler aufweisen, so dass das Element mit dem vorhergehenden bzw. nachfolgenden Element über Verbindungsstellen bzw. Kontaktstellen im Bereich der Schlaufentäler des Elements und der Schlaufenköpfe des nachfolgenden Elements bzw. im Bereich der Schlaufenköpfe des Elements und der Schlaufentäler des vorhergehenden Elements derart fest verbunden ist, dass eine einstückige, tubuläre bzw. zylinderförmige Wandstruktur entsteht. Weiter weist der erfindungsgemäße Stent im Bereich der Verbindungsstellen bzw. Kontaktstellen zwischen Schlaufentälern und Schlaufenköpfen spitze Winkel ϕ auf. Dieser Winkel ϕ wird gebildet aus den an den Verbindungsstellen zusammentreffenden Schlaufenkurven, und zwar aus den Tangenten der Kurven. Typischerweise sind diese Winkel kleiner als 45°, 40°, 35°, 30°, insbesondere kleiner als 20°, wobei kleiner 15° sowie 10° besonders bevorzugt ist, bezogen auf den expandierten Stent.

Unter Schlaufe wird hierbei eine einfache, im Wesentlichen offene Schlaufe verstanden, wie sie beispielsweise bei Tauwerken, wo diese Schlaufe Bucht heißt, verwendet wird. Die Elemente können hierbei eine Vielzahl von aneinander liegenden, benachbarten und miteinander fest verbundenen Gliedern darstellen, wobei diese Glieder ringförmig bzw. im Wesentlichen radial um die longitudinale Achse des Stent angeordnet sind. Das Element kann jedoch auch als eine Helix, eine Wendel, eine Spirale, eine Schraube oder als ein Teilabschnitt der genannten Strukturen ausgebildet sein. Hierbei kann eine Windung bzw. ein Umlauf um die radiale Achse ein Element darstellen. Eine Windung bezeichnet hierbei ein (Kreis-)Durchgang einer Helix, Wendel, Spirale oder Schraube. Die Struktur kann jedoch auch als eine Doppelhelix ausgebildet sein. In diesem Fall stellt jeweils eine Helix ein Element dar.

Im Sinne der Erfindung wird vorwiegend von einem Stent im nichtexpandierten Zustand ausgegangen. Dies schließt jedoch nicht aus, dass der erfindungsgemäße Stent nicht auch in einem expandierten oder teilweise expandierten Zustand die im Folgenden bzw. o.g. genannten beschriebenen Merkmale aufweist. Jedoch weist der erfindungsgemäße Stent, welcher für den Einsatz in röhrenförmigen Hohlorganen geeignet sein soll, eine dementsprechende Form auf, damit dieser überhaupt in das entsprechende Hohlorgan eingebracht werden kann. Ob der erfindungsgemäße Stent dann weiter expandiert wird oder werden kann, nur der Stabilität des Hohlorgans dienen soll oder für das Offenhalten des Hohlorgans sorgt, hängt von dem zu erreichenden Ziel der Behandlung bzw. der Therapie ab.

Überraschenderweise hat sich gezeigt, dass aufgrund der Struktur des erfindungsgemäßen Stents dieser an Biegestellen bzw. beim Biegen nicht einknickt.

Erfindungsgemäß ist es ebenfalls möglich, den erfindungsgemäßen Stent auch bei einem falschen oder ungenauen Ablegen im Gefäß mittels eines Katheters oder ähnlichem wieder in den Katheter bzw. die Schleuse des Katheters einzuziehen. Hierdurch ist die Implantation des erfindungsgemäßen Stents fehlerunanfälliger und einfacher zu realisieren.

Der erfindungsgemäße Stent wird vorteilhaft aus einem einzigen tubulären Materialstück hergestellt, vorteilhafterweise durch das Schneiden mittels Laser-Techniken. Hierdurch entstehen völlig glatte, radial nach außen gerichtete (murale) Oberflächen bzw. zur Innenseite des Stents gerichtete (luminale) Innenflächen. Durch die völlig glatte Innenfläche treten turbulente Strömungen, welche durch Ablagerungen an Unebenheiten bedingt werden, praktisch nicht auf, so dass sich stattdessen eine gewünschte laminare Strömung innerhalb des Stents einstellt. Auch werden durch die völlig glatte Oberfläche Traumen an der Gefäßwand nahezu vermieden.

Der erfindungsgemäße Stent weist an den Verbindungsstellen jeweils einen im Wesentlichen parallel zur longitudinalen Richtung verlaufenden Steg S₁ und einen im Wesentlichen parallel zur radialen Richtung verlaufenden Steg S₃ auf. Weiter weist der erfindungsgemäße Stent im Bereich der Schlaufenlinien zwischen den Schlaufenköpfen und den Schlaufentälern einen Steg S₂ auf.

Die erfindungsgemäßen Verbindungsstellen bzw. Kontaktstellen sind hierbei nicht zu verwechseln mit Verbindern wie sie aus dem Stand der Technik bekannt sind. Die Verbinder aus dem Stand der Technik weisen immer in longitudinaler Richtung des Stents eine gewisse Länge auf, so dass beispielsweise zwischen Schlaufenköpfen und in longitudinaler Richtung gegenüberliegenden Schlaufentälern immer ein Abstand vorhanden ist. Im Gegensatz hierzu weisen die erfindungsgemäßen Verbindungsstellen bevorzugt keinen solchen Abstand auf, so dass die Schlaufenköpf und die in longitudinaler Richtung gegenüberliegenden Schlaufentäler hier in einem direkten Kontakt stehen oder sich sogar überlagern.

Im weiteren Verlauf, radial weg von den Verbindungsstellen, verlaufen die Schlaufen bevorzugt, jedoch im Wesentlichen rund bzw. gebogen. Dies schließt jedoch einen anderen Verlauf der Schlaufen nicht aus. Erfindungsgemäß weist S₁ mindestens die Breite auf, welche ein Steg S₂ im Bereich der Schlaufenlinie zwischen dem Schlaufenkopf und dem Schlaufental aufweist. Erfindungsgemäß weist der Steg S₁ eine kürzere Länge als der Steg S₃ auf, wobei S₁ höchstens 1 bzw. ¾ mal so lang, bevorzugt höchstens 2/3 bzw. halb mal so lang, besonders bevorzugt höchstens 1/3 mal so lang wie S₃ ist. Dabei ist eine Länge von S₁ besonders bevorzugt, welche höchstens ¼ mal so lang wie die von S₃ ist. Bevorzugt ist das Verhältnis von S₂:S₁ maximal 1:2,5, insbesondere maximal 1:2,3 bzw. 1:2,1, wobei maximal 1:2,1 insbesondere maximal 1:2 besonders bevorzugt ist, mindestens jedoch 1:1,8, bevorzugt mindestens 1:1,5, besonders bevorzugt ein Verhältnis von mindestens 1:1.

Der erfindungsgemäße Stent weist im Bereich der Verbindungsstellen zwischen Schlaufentälern und Schlaufenköpfen spitze Winkel ϕ auf, die bei zunehmender, kontinuierlicher Expansion oder Aufdehnung des Stents ein geringeres Winkelmaß annehmen. D.h. bei Aufdehnung des Stents in radialer Richtung nach außen hin wird der Winkel ϕ immer kleiner und kann bei maximaler Aufdehnung einen Zustand >= 0 annehmen.

Durch die erfindungsgemäße Anordnung verkleinern sich bei Aufdehnung des Stents Winkel, welche ihre Ansatzpunkte an der Schlaufenlinie im Bereich zwischen dem Schlaufenkopf und dem Schlaufental haben, kontinuierlich. Dem Stent kann somit je nach Bedarf des Arztes bzw. der Anwendung entweder mehr Flexibilität und eine höhere Radialkraft wie dies bei einer großen Aufdehnung der Fall wäre, oder weniger Flexibilität mit geringerer Radialkraft wie dies beispielsweise bei einer kleinen Aufdehnung der Fall wäre, verliehen werden. Das Design oder die Struktur des Stents muss hierfür jedoch nicht geändert werden.

Vorteilhaft an diesen Ausbildungen des erfindungsgemäßen Stents ist zudem, dass beim Aufdehnen oder Expandieren, d. h. bei einer Vergrößerung des Durchmessers des Hohlraumes in radialer Richtung, der Stent an den Verbindungsstellen nicht einreißt, wie dies bei Stents aus dem Stand der Technik geschehen kann, da dort die radiale Zugkraft hauptsächlich im Bereich der Verbinder auftritt und nicht wie beim erfindungsgemäßen Stent über die im Wesentlichen runde bzw. gebogene Struktur mit jedoch spitzen Winkeln im Bereich der Verbindungsstellen mit aufgefangen wird. Dadurch wird die mechanische Beanspruchung in diesem Bereich beim gesetzten Stent sogar noch verringert, was zur Erhöhung der Stabilität führt.

Auch kann durch diese Struktur des erfindungsgemäßen Stents ein Abknicken im Bereich der Verbindungsstellen verhindert werden, da hier im Gegensatz zum Stand der Technik eben keine sog. Verbinder vorhanden sind, welche bedingt durch ihre Länge in longitudinaler Richtung bei Biegung des Stents einknicken können.

Prinzipiell ist es so, dass je mehr Schlaufen innerhalb eines Elementes radial ausgebildet werden, die Längenabnahme (Verkürzung) bei einem longitudinalen Zug umso kürzer ist, die Radialkraft jedoch umso größer ist. Jedoch wird hierbei der Stent unflexibler. Das Gegenteil ist der Fall, wenn weniger Schlaufen radial angelegt sind. Hier ist die Radialkraft des Stents geringer, jedoch wird dieser insgesamt flexibler. Letztlich wird jedoch durch diese Art der Ausbildung des erfindungsgemäßen Stents bei zumindest einem gleichmäßigen Zug in radialer bzw. longitudinaler Richtung keine Gefäßschädigung hervorgerufen, da der erfindungsgemäße Stent die Krafteinwirkung eines Zuges sowohl in radialer als auch in longitudinaler Richtung an den Verbindungsstellen minimiert. Erfindungsgemäß sind die Elemente aus mindestens 3 Schlaufen mit jeweils 3 Schlaufenköpfen und -tälern gebildet, wobei mindestens 4, insbesondere 5 bevorzugt ist. Die Maximalanzahl der Schlaufen beträgt vorteilhafterweise maximal 10 bzw. 8 Schlaufen, wobei maximal 7 Schlaufen und insbesondere maximal 6 Schlaufen bevorzugt sind.

Der erfindungsgemäße Stent ist bevorzugt so ausgebildet, dass die Elemente im Bereich der Schlaufenköpfe ein Maximum und im Bereich der Schlaufentäler ein Minimum aufweisen, wobei von Maximum zu Maximum die Schlaufen desselben Elements eine Länge λ in im Wesentlichen radialer Richtung y aufweisen und wobei das Schlaufenminimum bei A/2 oder einem ungeradzahligen Vielfachen davon liegt.

Vorteilhaft weist der erfindungsgemäße Stent im Bereich der Schlaufenköpfe bzw. -täler eines Elements, die Schlaufenköpfe bzw. -täler eine Ausdehnung b bzw. b' auf. Die Schlaufen im Bereich zwischen benachbarten Schlaufenköpfen bzw. -tälern eines Elements weisen hierbei eine Ausdehnung a bzw. a' auf, wobei a bzw. a' maximal gleich der Ausdehnung b bzw. b' ist, bevorzugt jedoch a bzw. a' größer 0 und kleiner b bzw. b' ist und besonders bevorzugt a bzw. a' gleich 0 ist, hier jedoch die benachbarten Schlaufenköpfe bzw. -täler eines Elements nicht fest verbunden vorliegen, d.h. bei einer Expansion des Stent a bzw. a' größer 0 werden kann. Die Ausdehnungen b, b', a und a' verlaufen bevorzugt bei einer ringförmigen bzw. helikalen Form der Elemente im Wesentlichen parallel zur radialen Richtung jedoch entlang der ringförmigen bzw. helikalen Form.

In einer weiteren Ausführungsform des erfindungsgemäßen Stent weist die Ausdehnung b bzw. b' einen Mittelpunkt m bzw. m' auf. Hierbei liegt der Mittelpunkt m bzw. m' der Ausdehnung b bzw. b' bevorzugt auf der Normalen N bzw. N', welche orthogonal zur Tangente des Schlaufenmaximums bzw. -minimums verläuft.

Erfindungsgemäß ist jedoch auch eine andere Formgebung der Schlaufe des erfindungsgemäßen Stents möglich. Die Schlaufen können beispielsweise mäanderförmig, Ω-förmig oder U-förmig ausgebildet sein. Erfindungsgemäß können die Schlaufen auch eine positive bzw. negative Neigung hin zur radialen Richtung y aufweisen. Ebenfalls können die Schlaufen einen teilweisen oder vollständigen gezackten, gewellten, aus einzelnen geraden Abschnitten gebildeten, glatten und/oder sonstigen Schlaufenlinienverlauf aufweisen.

Der erfindungsgemäße Stent kann selbstexpandierend bzw. nicht selbstexpandierend ausgestaltet sein. Die Expansion eines nicht selbstexpandierenden, erfindungsgemäßen Stents erfolgt üblicherweise mittels eines Ballonkatheters, der zuvor in das Innere des Stents eingeführt wurde. Vorteilhafterweise ist der erfindungsgemäße Stent jedoch selbstexpandierend bzw. selbstexpandierbar. Hierbei weist der erfindungsgemäße Stent vor der Implantation eine geringere radiale Ausdehnung auf und expandiert während bzw. nach der Implantation auf eine größere radiale Ausdehnung.

In einer weiteren vorteilhaften Ausführungsform ist der Stent aus einem Formgedächtnismaterial bzw. aus einer Formgedächtnislegierung (englisch: shape memory alloy, Abkürzung: SMA) gebildet. Formgedächtnismaterial wurde bereits für medizinische Implantate in verschiedene Anwendungen eingesetzt, so zum Beispiel auch für Stents zur Stabilisierung von Arterien. Hierbei ist es möglich, den Stent in komprimierter Form in ein Blutgefäß einzuführen, wo dieser sich dann bei Kontakt mit dem körperwarmen Blut in eine wirksame Form entfaltet bzw. expandiert. Insbesondere wird in einer zweckmäßigen Ausführungsform des erfindungsgemäßen Stents Nitinol, eine Nickel-Titan-Legierung, verwendet. Jedoch sind auch andere Legierungen denkbar, wie beispielsweise NiTiCu (Nickel-Titan-Kupfer), CuZn (Kupfer-Zink), CuZnAl (Kupfer-Zink-Aluminium), CuAlNi (Kupfer-Aluminium-Nickel), FeNiAl (Eisen-Nickel-Aluminium), FeMnSi (Eisen-Mangan-Silizium) und/oder ZnAuCu (Zink-Gold-Kupfer). Ebenfalls kann der erfindungsgemäße Stent auch aus anderen Materialien wie Metall, Kunststoff und/oder einer Verbindung der in diesem Absatz genannten Materialien hergestellt sein.

In einer weiteren vorteilhaften Ausgestaltung des erfindungsgemäßen Stents ist dieser röntgenopak ausgestaltet bzw. weist dieser mindestens eine röntgenopake Markierung auf. Dabei kann diese Markierung außerhalb und/oder innerhalb des erfindungsgemäßen Stents liegen. Durch die Markierung kann eine bessere Visualisierung des Stents während bzw. nach der Implantation gewährleistet werden.

Weiter kann in einer zusätzlichen Ausgestaltung des erfindungsgemäßen Stents der Stent Kopplungselemente an den longitudinalen, axialen Enden aufweisen, über welche mehrere Stents miteinander reversibel, direkt oder indirekt über beispielsweise Abstandshalter verbunden sind. Dies hat den Vorteil, dass mehrere Stents am gleichen Implantationsort oder an verschiedenen Implantationsorten innerhalb einer Implantation abgelegt werden können, wodurch beispielsweise die Operationszeit verringert werden kann.

Weiter kann der erfindungsgemäße Stent, obwohl dies nicht nötig ist, wenn er aus einem Stück geschnitten wird, an mindestens einer der Oberflächen und/oder an mindestens einem der longitudinalen, axialen Enden elektroplatiert, elektropoliert und/oder mechanisch poliert sein.

Üblicherweise werden Stenosen mittels eines Ballonkatheters dilatiert, um anschließend einen Stent setzen zu können. Derartige Dilatierungen führen zu Gefäßschädigungen, die von vielen Anwendern (Ärzten) nicht gewünscht werden. Durch den erfindungsgemäßen Stent ist es jedoch möglich, diesen zu setzen, ohne dass zuvor die Stenose mittels eines Ballons dilatiert wird. Durch die durch den erfindungsgemäßen Stent erhöhte, nach außen (mural) wirkende Radialkraft ist es nun möglich, den erfindungsgemäßen Stent in einer Stenose abzulegen und die Stenose über mehrere Tage hinweg ausschließlich durch den erfindungsgemäßen Stent nach außen zu erweitern bzw. zu beseitigen. Dies hat wiederum zur Folge, dass sich die Operationszeit verringert und Gefäßschädigungen, welche durch Dilatierungen mittels Ballonkatheter hervorgerufen werden, vermieden werden können.

Der erfindungsgemäße Stent eignet sich nicht nur für arterielle Anwendungen, sondern auch für venöse Anwendungen. Bei venösen Stents ist es so, dass diese oft und aufgrund der Architektur dieser Gefäße unterschiedliche Durchmesser aufweisen müssen. Nach dem Stand der Technik wurde dies beispielsweise dadurch gelöst, dass die einzelnen Elemente unterschiedlich groß ausgebildet wurden mit der Folge, dass beim Übergang von einem geringeren Durchmesser zu einem größeren Durchmesser Bereiche an den Verbindungsstellen der einzelnen Elemente in die Blutbahn hineinragen, was wiederum zur Ausbildung von nicht linearen Strömungen und damit verbundenen Ablagerungen führen kann.

In einer weiteren vorteilhaften Ausführungsform des erfindungsgemäßen Stents bilden jeweils zwei benachbarte Elemente ein Elementenpaar und weisen dabei eine Länge L in longitudinaler Richtung x auf. Die Länge L entspricht hierbei maximal 100%, jedoch minimal 21%, bevorzugt 75% und besonders bevorzugt 50% des Innendurchmessers des Elementenpaares im nicht-expandierten Zustand des Stents.

In einer weiteren vorteilhaften Ausführungsform des erfindungsgemäßen Stents ist die Länge L eines Elementenpaares gegenüber den in longitudinaler Richtung x benachbarten Elementenpaaren jeweils sukzessive verringert oder sukzessive vergrößert. Im Falle eines helikalen Elementes kann das Element auch innerhalb dieses Elements sukzessiv vergrößerte bzw. verringerte Schlaufen aufweisen. Werden große Schlaufen ausgebildet, dann entstehen beim Dilatieren große Radien. Somit sind langsame kontinuierliche Übergänge von großen zu kleineren Durchmessern bzw. umgekehrt möglich. Diese Übergänge weisen jedoch keine Stellen auf, die in die die Blutbahn hineinragen. Insbesondere bei kleinen Gefäßdurchmessern werden hohe Radialkräfte benötigt, welche sich insbesondere mit kleinen Schlaufen gemäß der erfindungsgemäßen Stentstruktur sehr gut realisieren lassen.

Mittels des erfindungsgemäßen Stents werden dünne, gleichförmig ausgebildete Verbindungsstellen bereitgestellt, welche das Innenvolumen des Gefäßes nahezu nicht verändern. Dabei besitzt der erfindungsgemäße Stent dennoch eine hohe, nach außen gerichtete Radialkraft, die es ermöglicht, den Gefäßdurchmesser über die gesamte Länge des erfindungsgemäßen Stent nach außen drückend zu vergrößern und/oder stabil zu halten.

Nachfolgend wird die Erfindung anhand von Ausführungsbeispielen in Zeichnungen näher beschrieben. Die Erläuterungen sind lediglich beispielhaft und schränken den allgemeinen Erfindungsgedanken nicht ein.

Es zeigen:
Fig. 1 eine schematische Darstellung eines Stents nach dem Stand der Technik mit einem starren Design, sogenannten Closed Cell Design;
Fig. 2 in einer schematischen Darstellung einen Stent nach dem Stand der Technik mit teilweise flexiblem Design, sogenanntem Open Cell Design;
Fig. 3 in einer schematischen Darstellung eine Ausführungsform des erfindungsgemäßen Stents;
Fig. 4 in einer schematischen Darstellung einen Ausschnitt einer Ausführungsform des erfindungsgemäßen Stents;
Fig. 5a bis 5c jeweils in einer schematischen Darstellung einen Ausschnitt einer Ausführungsform des erfindungsgemäßen Stents bei verschiedenen Zuständen der Ausdehnungen b, b', a und a';
Fig. 6 in einer schematischen 3D-Darstellung einen Ausschnitt einer Ausführungsform des erfindungsgemäßen Stents;
Fig. 7 jeweils in schematischen Darstellungen einen Ausschnitt sowie einen Detaillausschnitt des Winkels ϕ einer Ausführungsform des erfindungsgemäßen Stents bei verschiedenen Zuständen B, C, D, und E der Aufdehnung des Stents.

Fig. 1 zeigt einen Stent nach dem Stand der Technik im sogenannten starren bzw. Closed Cell Design. Hierbei sind mehrere Elemente 300, 310 nacheinander in longitudinaler Richtung x angeordnet. Dabei ist ein Element 300 kontinuierlich über die gesamte Länge in radialer Richtung y mittels Verbinder 220 mit dem nachfolgenden Element 310 verbunden.

Fig. 2 zeigt ebenfalls einen Stent nach dem Stand der Technik, wobei dieser teilweise ein flexibles, sogenanntes Open Cell Design aufweist. Bei diesem Design ist ein Element 300 in radialer Richtung y über Verbindern 220 mit einem in longitudinaler Richtung x benachbarten Element 310 verbunden. Jedoch besteht hier keine durchgängige Verbindung in radialer Richtung y, sondern es bestehen Bereiche, in welchen keine Verbindung zwischen den Elementen 300 bzw. 310 vorliegen.

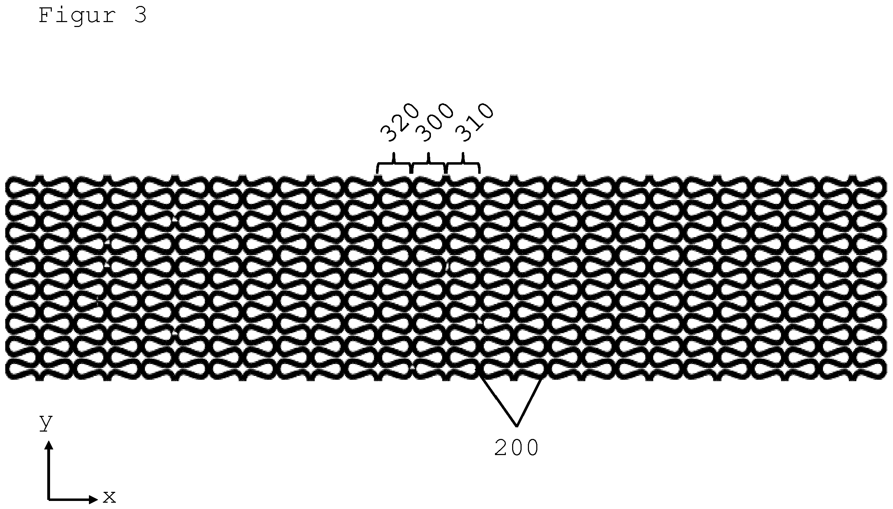

Fig. 3 zeigt eine Ausführungsform des erfindungsgemäßen Stents, wobei ein Element 300 mit in longitudinaler Richtung x vorhergehenden Element 320 bzw. nachfolgendem Element 310 über Verbindungsstellen 200 fest verbunden ist. Dabei weisen die Elemente 300, 310, 320 in radialer Richtung y die Form von Schlaufen mit Schlaufenköpfen und Schlaufentälern auf.

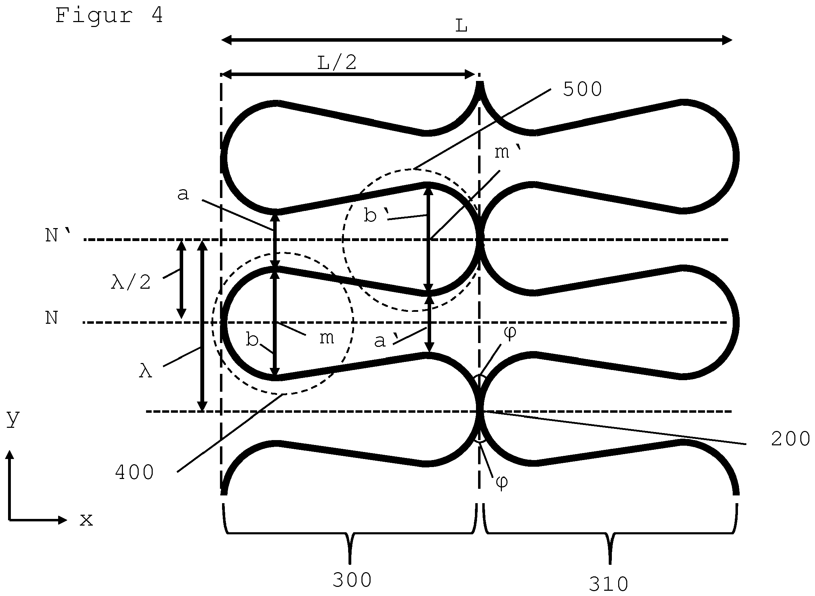

Fig. 4 zeigt einen schematischen Detailausschnitt einer Ausführungsform des erfindungsgemäßen Stents, wobei ein Element 300 und sein in longitudinaler Richtung x nachfolgendes, benachbartes Element 310 dargestellt ist. Das Element 300 weist hierbei Schlaufen mit Schlaufenköpfen 400 und Schlaufentälern 500 auf. Das Element 300 weist dabei eine Schlaufenlänge λ von einem Schlaufenkopf zu einem in radialer Richtung y benachbarten Schlaufenkopf auf, wobei hier jeweils das Schlaufental bei A/2 liegt. Weiter weist das Element 300 von den Schlaufenköpfen zu den Schlaufentälern in longitudinaler Richtung x eine Länge L/2 auf. Das Elementenpaar 300, 310 weist dabei eine Länge L in longitudinaler Richtung x auf. Das Element 300 ist gegenüber dem nachfolgenden Element 310 um eine halbe Schlaufenlänge A/2 verschoben, derart, dass seine Schlaufentäler mit den Schlaufenköpfen des nachfolgenden Elements 310 über Verbindungsstellen 200 fest verbunden sind. Weiter ist im Bereich der Schlaufenköpfe bzw. -täler des Elements 300 eine Ausdehnung b bzw. b' mit einem Mittelpunkt m bzw. m' dargestellt. Der Mittelpunkt m bzw. m' der Ausdehnung b bzw. b' liegt in dieser Ausführungsform auf der Normalen N bzw. N', welche orthogonal zur Tangente des Maximums bzw. Minimums des Schlaufenkopfes bzw. -tales verläuft.

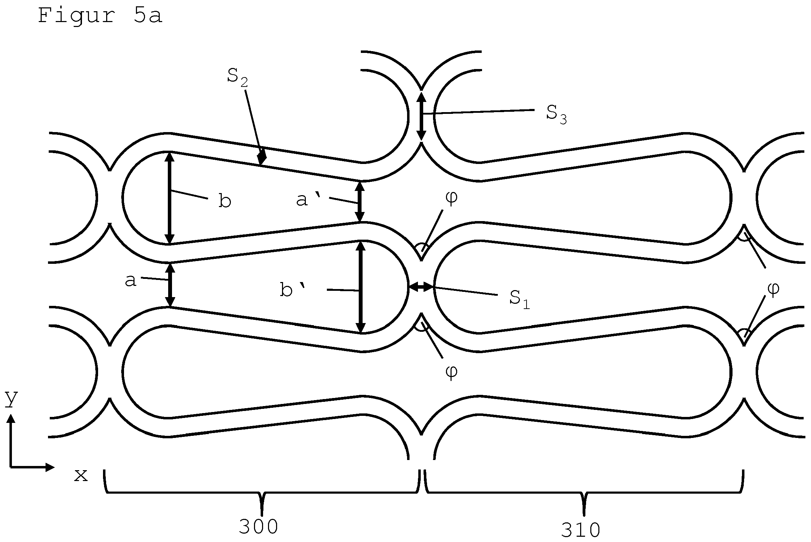

Fig. 5a zeigt eine Ausführungsform des erfindungsgemäßen Stent in einem Zustand, bei welchem die Ausdehnung a bzw. a' größer 0, jedoch kleiner als b bzw. b' ist. Mit Zustand ist hier ein nicht-expandierter Zustand gemeint, in dem der Stent noch weiter aufgedehnt, d.h. sein Durchmesser noch in radialer Richtung nach außen vergrößert werden kann. Dargestellt sind weiter ein Steg S₁, welcher parallel zur longitudinaler Richtung x verläuft, und ein Steg S₃, welcher parallel zur radialen Richtung y ausgebildet ist. Weiter ist ein Steg S₂ dargestellt, welcher der Breite der Schlaufenlinie im Bereich zwischen dem Schlaufenkopf und dem Schlaufental entspricht. Hierbei ist es so, dass S₁ mindestens die Breite aufweist, die S₂ aufweist, und dass der Steg S₁ eine kürzere Länge als der Steg S₃ aufweist. Weiter weist der Stent im Bereich der Verbindungsstellen zwischen Schlaufentälern und Schlaufenköpfen spitze Winkel ϕ auf, welche bei Expansion des Stents ein geringeres Winkelmaß annehmen. Der Stent weist jedoch keine sog. Verbinder auf, sodass an den Verbindungsstellen zwischen Schlaufentälern und Schlaufenköpfen keine Abstände (Verbinder) vorhanden sind. Vielmehr überlagern sich an den erfindungsgemäßen Verbindungsstellen die Schlaufentäler und die Schlaufenköpfe.

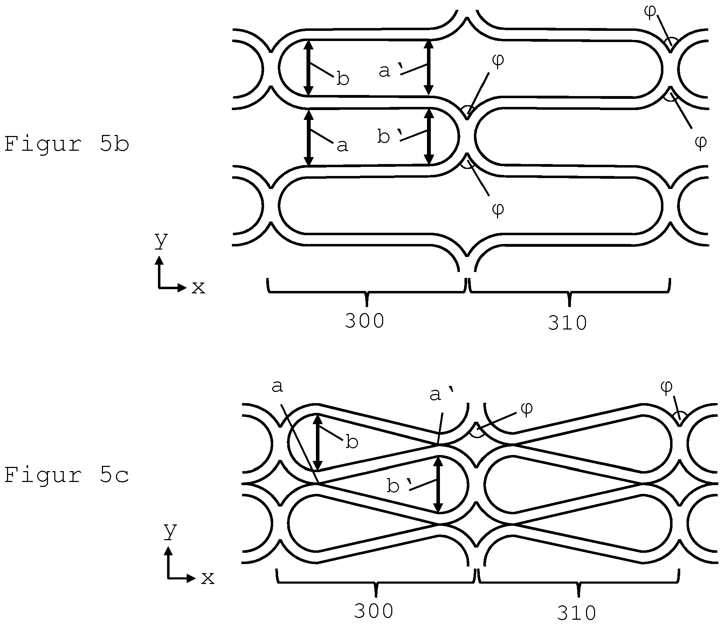

Fig. 5b zeigt eine Ausführungsform des erfindungsgemäßen Stents in einem Zustand, bei welchem die Ausdehnung a bzw. a' gleich der von b bzw. b' ist. Mit Zustand ist hier ein nicht-expandierter Zustand gemeint, in dem der Stent noch weiter aufgedehnt, d.h. sein Durchmesser noch in radialer Richtung nach außen vergrößert werden kann.

Fig. 5c zeigt eine Ausführungsform des erfindungsgemäßen Stents in einem Zustand, bei welchem die Ausdehnung a bzw. a' gleich 0 und b bzw. b' größer 0 ist. Hier sind jedoch die benachbarten Schlaufenköpfe bzw. -täler im Bereich von a bzw. a' eines Elements nicht fest miteinander verbunden, d.h. bei einer Expansion des Stents kann a bzw. a' wieder größer 0 werden. Mit Zustand ist hier ein nicht-expandierter Zustand gemeint, in dem der Stent noch weiter aufgedehnt, d.h. sein Durchmesser noch in radialer Richtung nach außen vergrößert werden kann.

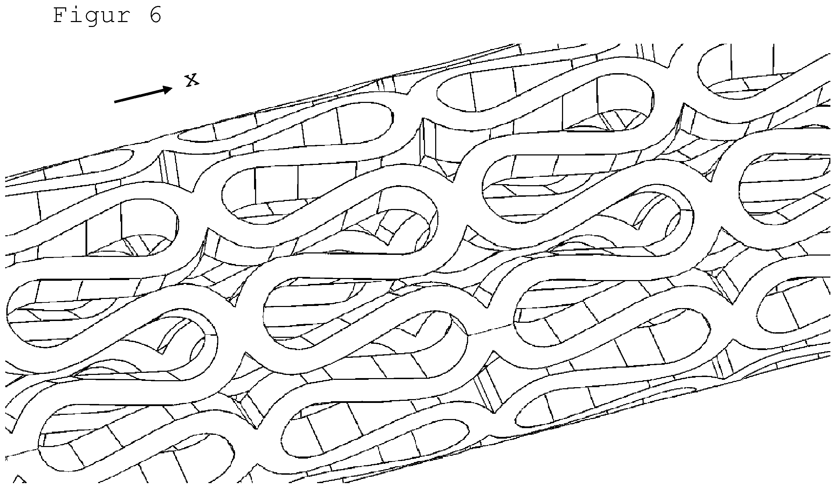

Fig. 6 zeigt einen Detailausschnitt einer Ausführungsform des erfindungsgemäßen Stents in einer schematischen 3D-Darstellung.

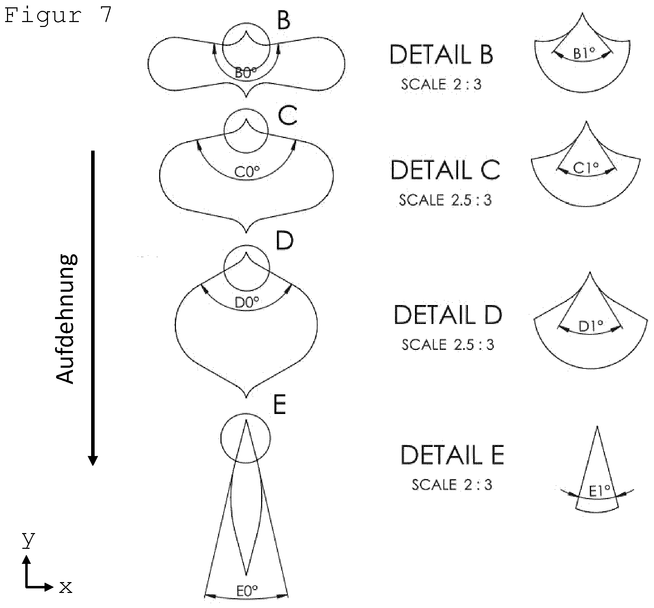

Fig. 7 zeigt eine Ausführungsform des erfindungsgemäßen Stents bei verschiedenen Aufdehnungszuständen. Die Zustände reichen hierbei von B wenig bis gar nicht aufgedehnt über C und D zu E, sehr weit aufgedehnt, wobei die Aufdehnungszustände im Verhältnis E>D>C>B stehen. Der spitze Winkel ϕ, hier je nach Aufdehnungszustand als B1 bis E1 dargestellt, verkleinert sich hierbei bei Aufdehnung von B nach E im Verhältnis B1>C1>D1>E1. E1 kann bei maximaler Aufdehnung einen Wert von E1>=0 annehmen. Auch der Winkel, welcher seine Ansatzpunkte an der Schlaufenlinie im Bereich zwischen dem Schlaufenkopf und dem Schlaufental hat und mit B0 bis E0 bezeichnet ist, verkleinert sich bei Aufdehnung von B nach E im Verhältnis B0>C0>D0>E0. Dem Stent kann so entweder mehr Flexibilität und eine höhere Radialkraft wie dies bei Zustand E der Fall wäre, oder weniger Flexibilität mit geringerer Radialkraft wie dies beispielsweise bei Zustand C der Fall wäre, verliehen werden. Das Design oder die Struktur des Stents muss hierfür jedoch nicht geändert werden.

### Bezugszeichenliste

- 200: Verbindungsstelle
- 210: nicht verbundene Stelle
- 220: Verbinder
- 300: Element
- 310: nachfolgendes Element
- 320: vorhergehendes Element
- 400: Bereich des Schlaufenkopfes
- 500: Bereich des Schlaufentales
- λ: Schlaufenlänge
- λ/2: halbe Schlaufenlänge
- ϕ: spitzer Winkel
- B1: spitzer Winkel ϕ bei Ausdehnungszustand B
- C1: spitzer Winkel ϕ bei Ausdehnungszustand C
- D1: spitzer Winkel ϕ bei Ausdehnungszustand D
- E1: spitzer Winkel ϕ bei Ausdehnungszustand E
- B0: Winkel bei Ausdehnungszustand B
- C0: Winkel bei Ausdehnungszustand C
- D0: Winkel bei Ausdehnungszustand D
- E0: Winkel bei Ausdehnungszustand E
- B: Ausdehnungszustand des Stents
- C: Ausdehnungszustand des Stents
- D: Ausdehnungszustand des Stents
- E: Ausdehnungszustand des Stents
- a: Ausdehnung im Bereich zwischen benachbarten Schlaufenköpfen eines Elements
- a': Ausdehnung im Bereich zwischen benachbarten Schlaufentälern eines Elements
- b: Ausdehnung im Bereich eines Schlaufenkopfes
- b': Ausdehnung im Bereich eines Schlaufentales
- L: Länge eines Elementenpaares in longitudinaler Richtung
- L/2: Länge eines Elements in longitudinaler Richtung
- m: Mittelpunkt der Ausdehnung b
- m': Mittelpunkt der Ausdehnung b'
- N: Normale, welche orthogonal zur Tangente eines Schlaufenminimums ist
- N': Normale, welche orthogonal zur Tangente eines Schlaufenminimums ist
- S₁: Steg im Bereich der Verbindungsstelle, welcher im Wesentlichen parallel zur longitudinalen Richtung verläuft
- S₂: Steg im Bereich der Schlaufenlinie zwischen dem Schlaufenkopf und dem Schlaufental
- S₃: Steg im Bereich der Verbindungsstelle, welcher im Wesentlichen parallel zur radialen Richtung verläuft
- x: longitudinale Richtung
- y: radiale Richtung

## Patentansprüche

1. Stent mit einem durch eine Wand begrenzten, durchgängigen, innenliegenden, tubulären bzw. zylinderförmigen Hohlraum, wobei die Wand um eine in longitudinaler Richtung (x) verlaufende Achse herum tubulär, insbesondere zylinderförmig ausgebildet ist und eine die Wand umlaufende Struktur aufweist, wobei die Struktur aus Elementen (300, 310, 320) gebildet ist, wobei die Elemente (300, 310, 320) aus Schlaufen gebildet sind, welche in radialer Richtung (y) um die longitudinale Achse herum angeordnet sind und alternierend angeordnete, zur longitudinalen Richtung (x) parallel verlaufende Schlaufenköpfe (400) und Schlaufentäler (500) aufweisen, wobei jeweils alle Elemente mit dem vorhergehenden bzw. nachfolgenden Elementen über Verbindungsstellen (200) im Bereich der Schlaufentäler der Elemente und der Schlaufenköpfe der nachfolgenden Elemente bzw. im Bereich der Schlaufenköpfe der Elemente und der Schlaufentäler der vorhergehenden Elemente derart fest verbunden sind, dass eine einstückige, tubuläre, insbesondere zylinderförmige Wandstruktur entsteht, **dadurch gekennzeichnet, dass** die Schlaufenköpfe und die in longitudinaler Richtung gegenüberliegenden Schlaufentäler in einem direkten Kontakt stehen oder sich überlagern und dass an den Verbindungsstellen (200) jeweils ein im Wesentlichen parallel zur longitudinalen Richtung (x) verlaufender Steg S₁, und jeweils ein im Wesentlichen parallel zur radialen Richtung (y) verlaufender Steg S₃, ausgebildet ist, wobei S₁ mindestens die Breite aufweist, welche ein Steg S₂ im Bereich der Schlaufenlinie zwischen dem Schlaufenkopf und dem Schlaufental aufweist und dass im Bereich der Verbindungsstellen (200) zwischen Schlaufentälern (500) und Schlaufenköpfen (400) spitze Winkel (ϕ) vorliegen, die ihre Ansatzpunkte an der Schlaufenlinie im Bereich zwischen dem Schlaufenkopf (400) und dem Schlaufental (500) haben.

2. Stent nach Anspruch 1, **dadurch gekennzeichnet, dass** die spitzen Winkel (ϕ) bei zunehmender, kontinuierlicher Expansion des Stents ein geringeres Winkelmaß annehmen.

3. Stent nach einem der vorhergehenden Ansprüche, **dadurch gekennzeichnet, dass** der Stent aus einem einzigen tubulären Materialstück hergestellt ist.

4. Stent nach einem der vorhergehenden Ansprüche, **dadurch gekennzeichnet, dass** Stent aus einem Metall, einer Legierung, einem Kunststoff, aus einem Formgedächtnismaterial, aus Nitinol oder einer Verbindung dieser Materialen gebildet ist.

5. Stent nach einem der vorhergehenden Ansprüche, **dadurch gekennzeichnet, dass** die Schlaufen mäanderförmig, Ωförmig oder U-förmig ausgebildet sind.

6. Stent nach einem der vorhergehenden Ansprüche, **dadurch gekennzeichnet, dass** die Schlaufen einen teilweisen oder vollständigen gezackten, gewellten, aus einzelnen geraden Abschnitten gebildeten oder glatten Schlaufenlinienverlauf aufweisen.

7. Stent nach einem der vorhergehenden Ansprüche, **dadurch gekennzeichnet, dass** der Steg S₁ eine kürzere Länge als der Steg S₃ aufweist, wobei S₁ höchstens 1 mal, höchstens ¾ mal, höchstens 2/3 mal, höchstens ½ mal, höchstens 1/3 mal oder höchstens ¼ mal so lang ist wie die Länge von S₃.

8. Stent nach einem der vorhergehenden Ansprüche, **dadurch gekennzeichnet, dass** der Stent röntgenopak ausgestaltet ist oder mindestens eine röntgenopake Markierung aufweist.

9. Stent nach einem der vorhergehenden Ansprüche, **dadurch gekennzeichnet, dass** der Stent an den longitudinalen, axialen Enden Kopplungselemente aufweist, über welche mehrere Stents miteinander, reversibel direkt oder indirekt über Abstandshalter verbunden sind.

10. Stent nach einem der vorhergehenden Ansprüche, **dadurch gekennzeichnet, dass** der Stent an mindestens einer der Oberflächen und/oder an mindestens einem der longitudinalen, axialen Enden elektroplatiert, elektropoliert und/oder mechanisch poliert ist.

11. Stent nach einem der vorhergehenden Ansprüche, **dadurch gekennzeichnet, dass** die Elemente (300, 310, 320) im Bereich der Schlaufenköpfe (400) ein Maximum und im Bereich der Schlaufentäler (500) ein Minimum aufweisen, wobei von Maximum zu Maximum desselben Elements die Schlaufen eine Länge λ in radialer Richtung (y) aufweisen und wobei das Schlaufenminimum bei λ/2 oder einem ungeradzahligen Vielfachen davon liegt.

12. Stent nach einem der vorhergehenden Ansprüche, **dadurch gekennzeichnet, dass** die Elemente (300, 310, 320) aus mindestens 3 Schlaufen mit jeweils 3 Schlaufenköpfen und -tälern gebildet sind.

13. Stent nach einem der vorhergehenden Ansprüche, **dadurch gekennzeichnet, dass** im inneren Bereich der Schlaufenköpfe bzw. -täler eines Elements, die Schlaufenköpfe bzw. -täler eine Ausdehnung b bzw. b' aufweisen, und dass die Schlaufen im Bereich zwischen benachbarten Schlaufenköpfen bzw. -tälern eines Elements (300) eine Ausdehnung a bzw. a' aufweisen, wobei a bzw. a' maximal gleich der Ausdehnung b bzw. b' ist, a bzw. a' größer 0 und kleiner b bzw. b' ist, oder a bzw. a' gleich 0 ist, wobei hier jedoch die benachbarten Schlaufenköpfe bzw. -täler eines Elements (300) nicht fest verbunden vorliegen.

14. Stent nach einem der vorhergehenden Ansprüche, **dadurch gekennzeichnet, dass** jeweils zwei benachbarte Elemente ein Elementenpaar bilden und eine Länge L in longitudinaler Richtung (x) aufweisen, wobei die Länge L maximal 100%, 75%, 50% oder minimal 21%des Innendurchmessers des Elementenpaares im nicht-expandierten Zustand des Stents entspricht.

15. Stent nach Anspruch 13, **dadurch gekennzeichnet, dass** die Länge L eines Elementenpaares gegenüber den in longitudinaler Richtung (x) benachbarten Elementenpaaren jeweils sukzessive verringert oder sukzessive vergrößert ist.

## Claims

1. A stent with a continuous, interior, tubular or cylindrical cavity bounded by a wall, wherein the wall is configured to be tubular, in particular cylindrical, extending about an axis running in a longitudinal direction (x) and comprises a structure running about the wall, wherein the structure is formed from elements (300, 310, 320) and the elements (300, 310, 320) are formed from loops, which are arranged in a radial direction (y) about the longitudinal axis, and they have loop peaks (400) and loop valleys (500) arranged in an alternating manner and running parallel to the longitudinal direction (x), wherein all elements are firmly connected to preceding or respectively following elements by connection points (200) in the region of the loop valleys of the elements and the loop peaks of the following elements or respectively in the region of the loop peaks of the elements and the loop valleys of the preceding elements, such that a one-piece tubular, in particular cylindrical, wall structure is created, **characterized in that** the loop peaks and the loop valleys opposite to the loop peaks in the longitudinal direction stand in direct contact or overlap each other, and that a web S₁ running substantially parallel to the longitudinal direction (x) and a web S₃ running substantially parallel to the radial direction (y) is formed for each of the connection points (200), wherein S₁ has at least the width of a web S₂ in the region of the loop line between the loop peak and the loop valley and acute angles (ϕ) are present in the region of the connection points (200) between loop valleys (500) and loop peaks (400), wherein the acute angles (ϕ) have their starting points on the loop line in the region between the loop peak (400) and the loop valley (500).

2. The stent according to claim 1, **characterized in that** the acute angles (ϕ) take on a smaller angular dimension under increasing continual expansion of the stent.

3. The stent according to one of the preceding claims, **characterized in that** the stent is made from a single tubular piece of material.

4. The stent according to one of the preceding claims, **characterized in that** the stent is formed from a metal, an alloy, a plastic, a shape memory material, Nitinol, or a combination of these materials.

5. The stent according to one of the preceding claims, **characterized in that** the loops are formed meandering, Ω-shaped or U-shaped.

6. The stent according to one of the preceding claims, **characterized in that** the loops have a loop line which is partly or entirely jagged, wavy, formed from individual straight segments, or smooth.

7. The stent according to one of the preceding claims, **characterized in that** the web S₁ has a shorter length than the web S₃, S₁ being at most 1 time, at most %, at most 2/3, at most ½, at most 1/3, or at most % the length of S₃.

8. The stent according to one of the preceding claims, **characterized in that** the stent is radiologically opaque or at least has a radiologically opaque marking.

9. The stent according to one of the preceding claims, **characterized in that** the stent comprises coupling elements at the longitudinal, axial ends, by which several stents are reversibly joined together directly or indirectly by spacers.

10. The stent according to one of the preceding claims, **characterized in that** the stent is electroplated, electropolished and/or mechanically polished at least on one of the surfaces and/or at least at one of the longitudinal, axial ends.

11. The stent according to one of the preceding claims, **characterized in that** the elements (300, 310, 320) have a maximum in the region of the loop peaks (400) and a minimum in the region of the loop valleys (500), wherein the loops have a length λ in the radial direction (y) from maximum to maximum of the same element and wherein the loop minimum lies at λ/2 or an odd multiple thereof.

12. The stent according to one of the preceding claims, **characterized in that** the elements (300, 310, 320) are formed from at least 3 loops each with 3 loop peaks and valleys.

13. The stent according to one of the preceding claims, **characterized in that** in the inner region of the loop peaks or valleys of an element, the loop peaks or valleys have an extension b or b', respectively, and the loops in the region between neighboring loop peaks or valleys of an element (300) have an extension a or a', respectively, wherein a or a' is at most equal to the extension b or b', a or a' is larger than 0 and smaller than b or b', or a or a' is equal to 0, but the neighboring loop peaks or valleys of an element (300) are not firmly connected here.

14. The stent according to one of the preceding claims, **characterized in that** each time two neighboring elements form an element pair and have a length L in the longitudinal direction (x), wherein the length L corresponds at most to 100%, 75%, 50% or at least to 21% of the internal diameter of the element pair in the non-expanded condition of the stent.

15. The stent according to claim 13, **characterized in that** the length L of an element pair each time successively decreases or successively increases in relation to the neighboring element pairs in the longitudinal direction (x).

## Revendications

1. Stent à cavité continue, interne, tubulaire ou cylindrique délimitée par une paroi, la paroi étant tubulaire, notamment cylindrique, autour d'un axe s'étendant dans la direction longitudinale (x) et ayant une structure entourant la paroi, la structure étant formée d'éléments (300, 310, 320), les éléments (300, 310, 320) étant formés de boucles, qui sont disposées dans une direction radiale (y) autour de l'axe longitudinal et qui présentent des têtes de boucle (400) et des creux de boucle (500) disposés en alternance et s'étendant parallèlement à la direction longitudinale (x), tous les éléments étant reliés avec les éléments précédents ou suivants par l'intermédiaire de points de connexion (200) dans la zone des creux de boucle et des têtes de boucle des éléments suivants ou dans la zone des têtes de boucle et des creux de boucle des éléments précédents étant connectés de sorte qu'une structure de paroi bosselée, tubulaire, en particulier cylindrique, est formée, **caractérisée en ce que** les têtes de boucle et les creux de boucle opposés dans la direction longitudinale sont en contact direct ou se chevauchent, et qu'une traverse S₁ aux points de connexion (200) est essentiellement parallèle à la direction longitudinale (x), dans chaque cas une traverse S₃ est formée qui s'étend essentiellement parallèlement à la direction radiale (y), S₁ ayant au moins la largeur que présente une traverse S₂ dans la zone de la ligne de boucle entre la tête de boucle et le creux de boucle, et **en ce que** dans la zone des points de connexion (200) entre les creux de boucle (500) et les têtes de boucle (400), il existe des angles aigus (ϕ) dont les points de départ sont sur la ligne de boucle dans la zone entre la tête de boucle (400) et le creux de boucle (500).

2. Stent selon la revendication 1, **caractérisé en ce que** les angles aigus (ϕ) prennent une dimension angulaire plus petite à mesure que le stent se dilate de manière continue et progressive.

3. Stent selon l'une quelconque des revendications précédentes, **caractérisé en ce que** le stent est constitué d'une seule pièce de matériau tubulaire.

4. Stent selon l'une quelconque des revendications précédentes, **caractérisé en ce que** le stent est constitué d'un métal, d'un alliage, d'un plastique, d'un matériau à mémoire de forme, de nitinol ou d'un composé de ces matériaux.

5. Stent selon l'une quelconque des revendications précédentes, **caractérisé en ce que** les boucles sont en forme de méandre, en forme de Ω ou en forme de U.

6. Stent selon l'une quelconque des revendications précédentes, **caractérisé en ce que** les boucles présentent une ligne de boucle lisse, ondulée, partiellement ou complètement dentelée, formée de sections rectilignes individuelles.

7. Stent selon l'une quelconque des revendications précédentes, **caractérisé en ce que** la traverse S₁ a une longueur plus courte que la traverser S₃, où S₁ est au maximum 1 fois, au maximum 3/4 fois, au maximum 2/3 fois, au maximum 1/2 fois, au maximum 1/3 fois ou au maximum 1/4 fois plus longue que la traverse S₃.

8. Stent selon l'une quelconque des revendications précédentes, **caractérisé en ce que** le stent est radio-opaque ou possède au moins un marquage radio-opaque.

9. Stent selon l'une quelconque des revendications précédentes, **caractérisé en ce que** le stent présente des éléments de couplage aux extrémités longitudinales, axiales, par lesquels plusieurs stents sont reliés entre eux, de manière réversible, directement ou indirectement par des entretoises.

10. Stent selon l'une quelconque des revendications précédentes, **caractérisé en ce que** le stent est électroplaqué, électropoli et/ou poli mécaniquement sur au moins une des surfaces et/ou au moins une des extrémités longitudinales, axiales.

11. Stent selon l'une quelconque des revendications précédentes, **caractérisé en ce que** les éléments (300, 310, 320) ont un maximum dans la région des têtes de boucle (400) et un minimum dans la région des creux de boucle (500), dans lequel du maximum au maximum du même élément les boucles ont une longueur λ dans la direction radiale (y) et dans lequel le minimum de boucle est à À/2 ou un multiple impair de celui-ci.

12. Stent selon l'une quelconque des revendications précédentes, **caractérisé en ce que** les éléments (300, 310, 320) sont formés d'au moins 3 boucles ayant chacune 3 têtes de boucle et creux de boucle.

13. Stent selon l'une quelconque des revendications précédentes, **caractérisé en ce que** dans la région intérieure des têtes ou creux de boucle d'un élément, les têtes ou creux de boucle ont une extension b ou b', et que les boucles dans la zone entre les têtes ou creux de boucle adjacents d'un élément (300) ont une extension a ou a', où a ou a' est au plus égal à l'extension b ou b', a ou a' est supérieur à 0 et inférieur à b ou b', ou a ou a' est égal à 0, mais dont les têtes ou creux de boucle adjacents d'un élément (300) ne sont pas fermement connectés.

14. Stent selon l'une quelconque des revendications précédentes, **caractérisé en ce que** deux éléments adjacents forment chacun une paire d'éléments et ont une longueur L dans la direction longitudinale (x), la longueur L correspondant à un maximum de 100 %, 75 %, 50 % ou un minimum de 21 % du diamètre intérieur de la paire d'éléments à l'état non étendu du stent.

15. Stent selon la revendication 13, **caractérisé en ce que** la longueur L d'une paire d'éléments est successivement réduite ou successivement augmentée par rapport aux paires d'éléments adjacentes dans la direction longitudinale (x).
